# EUROPEAN PATENT APPLICATION

(11) **EP 2 644 598 A1**
(43) Date of publication of application: **02.10.2013**
(21) Application number: 12162419.1
(22) Date of filing: 30.03.2012
(51) Int. Cl.: C07D 231/34

(54) **Method for the manufacture of heterocycles**

(71) Applicant: SOLVAY SA, 1120 Bruxelles (BE)
(72) Inventor: Januzems, Janis, 30559 Hannover (DE)
(74) Representative: Mross, Stefan P.M.

(57) **Abstract**

Heterocyclic compounds, e.g. alkyl 1-alkyl-3-halomethyl-pyrazole-4-carboxylates, can be manufactured using a hydrazinium-CO₂ compound, monosubstituted by an R¹ group, to introduce a =N-N(alkyl) group into the heterocycle. R¹ is an organic substituent selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group.

## Description

The present invention concerns a method for the manufacture of heterocycles.

Monosubstituted hydrazine, especially monoalkyl hydrazine, is a compound which can be used for the manufacture of, 1-substituted pyrazoles, e.g. 1-alkylpyrazoles as is described in US 2011/000962. Such pyrazoles are intermediates for fungicides.

Object of the present invention is to provide an improved method for the manufacture of heterocycles with an =N-N(R¹) group, e.g. for the manufacture of 3-haloalkylpyrazole-4-carboxylic acid derivates substituted in the C1 position by an R¹ group which is an organic substituent, especially an alkyl group.

Accordingly, the invention concerns a process for the manufacture of heterocyclic compounds having a heterocyclic ring with an =N-N(R¹) group in the ring wherein R¹ is an organic substituent wherein a hydrazinium-CO₂ compound, monosubstituted by an R¹ group, is applied to introduce the =N-N(R¹) group into the heterocyclic ring. The term "hydrazinium-CO₂ compound, monosubstituted by an R¹ group ","monosubstituted hydrazinium-CO₂ compound", "adduct of the monosubstituted hydrazine of the formula H₂N-NHR¹ and CO₂" and "monosubstituted hydrazinium-carboxylate compound" are used interchangeably in the frame of the present invention. The term "monosubstituted" in relation to the hydrazine always denotes "substituted by 1 R¹ group".

R¹ may be an organic substituent selected from the group consisting of C1 to C 12 alkyl ; C3 to C8 cycloalkyl ; C2 to C 12 alkenyl ; C2 to C 12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group.

R¹ preferably is C1 to C5 alkyl ; or C1 to C5 alkyl, substituted by at least one halogen atom, and more preferably, R¹ is methyl, ethyl, n-propyl, i-propyl, n-butyl, s-butyl, t-butyl or n-pentyl. Especially preferably, R¹ is methyl or ethyl, most preferably, methyl.

The monosubstituted hydrazinium-CO₂ compound may be provided by the reaction of CO₂ and the monosubstituted hydrazine of the formula H₂N-NH(R¹). It is an adduct of the monosubstituted hydrazine and CO₂. R₁ has the meaning given above ; R¹ is methyl, ethyl, n-propyl, i-propyl, n-butyl, s-butyl, t-butyl or n-pentyl. Especially preferably, R¹ is methyl or ethyl, most preferably, methyl. The monosubstituted hydrazine which is used as starting compound to provide the adduct with CO₂, has the general formula H₂N-NHR¹. Without being bound by any theory, it is assumed that it has a zwitterionic structure. It is assumed that the adduct corresponds to H₃N⁺-N (R¹)-C(O)O⁻ and/or H₂R¹N⁺-NH-C(O)O⁻. While the zwitterionic adduct corresponds to a 1:1 molar ratio of monosubstituted hydrazine:CO₂, a multitude of the zwitterionic compounds may form a 3-dimensional network which occludes additional monosubstituted hydrazine. Thus, when monosubstituted hydrazine and CO₂ are reacted in the absence of a solvent, a gel may form which contains the monosubstituted hydrazine:CO₂ in a global ratio of approximately 2:1 ; 1 mol of monosubstituted hydrazine is occluded in the network formed by the zwitterionic adduct of 1 mol of monosubstituted hydrazine and 1 mol of CO₂.

The adduct of monosubstituted hydrazine and CO₂ can be prepared by reacting the compounds neat or in a solvent.

Preferred solvents are organic solvents but liquid CO₂ (performed under pressure) or supercritical CO₂ may also be applied. If manufactured in supercritical CO₂, the molar ratio may be about 1:1, in organic solvents the molar ratio of the monosubstituted hydrazine:CO₂ is about 2:1.

Protic, aprotic, polar and nonpolar organic solvents may be used for the preparation of the adduct of monoalkylhydrazine and CO₂.

For example, aromatic solvents, e.g. toluene, are suitable. Alcohols are also suitable as solvent, e.g. monobasic alcohols and dibasic alcohols, for example, methanol, ethanol, n-propanol, isopropanol or glycol. Hydrocarbons can be applied, for example hydrocarbons with 3 to 10 carbon atoms, e.g. n-butane, 2-methylpropane, any isomers of pentane, e.g. n-pentane, any isomers of hexane, e.g. n-hexane, any isomers of heptane, e,g, n-heptane, and any mixtures thereof. Halohydrocarbons are suitable as solvent, e.g. saturated halohydrocarbons with 1 to 10 carbon atoms wherein halo denotes chlorine and fluorine. For example, dichloromethane, trichloromethane, tetrachloromethane, trichloroethane, any isomers of pentafluoropropane, e.g. 1,1,1,3,3-pentafluoropropane, the two isomers of heptafluoropropane, e.g. 1,1,1,2,3,3,3-heptafluoropropane, any isomers of pentafluorobutane, e.g. 1,1,1,3,3-pentafluorobutane, fluorosubstituted pentanes, e.g. decafluoropentane, and any mixtures thereof.

It was observed that in toluene, the adduct is formed as a viscous emulsion, in other solvents, e.g. in 1,1,1,3,3-pentafluorobutane, ethanol or hexane, the adduct is formed as a white, sometimes sticky suspension in the solvent.

The adduct, in the absence or in the presence of a solvent, in the form of an emulsion or suspension, can be used as intermediate in the manufacture of organic compounds having a (=N-N-R¹) group in the molecule. For example, it can be used as a substitute for monosubstituted hydrazine (not being an adduct with CO₂) to produce organic compounds having a (=N-N-R¹) group in the molecule. Often, the respective organic compounds are heterocyclic compounds.

Preferably, the adduct of monosubstituted hydrazine and CO₂ is used to prepare derivatives of 1-R¹-3-haloalkyl-pyrazole-4-carboxylic acid derivatives, especially alkyl 1-alkyl-3-halomethyl-pyrazole-4-carboxylates.

Accordingly, a preferred embodiment of the present invention concerns a method for the manufacture of 1-alkyl-3-haloalkyl-pyrazole-4-carboxylic acid derivatives of formula (I)

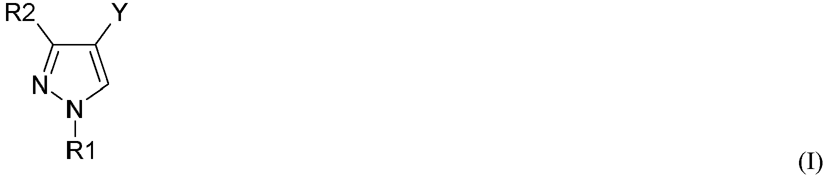

In which R¹ has the meaning given above ; R² is selected from C1 to C4 alkyl groups which may be substituted by one, two or three halogen atoms selected from the group consisting of F, Cl and Br or a CF₃ group ;

Y is selected from the group consisting of C(O)OR³, CN and C(O)NR⁴R⁵ wherein R³, R⁴ and R⁵ are independently of each other are selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁴ and R⁵ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine, preferably fluorine or chlorine ;
a) by reaction of a 2-acylated or 2-iminoalkylated acrylic acid derivative of the formula (II) 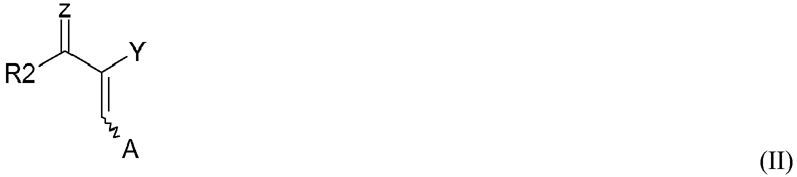 in which Z is selected from the group consisting of O, S and N⁺R¹⁰R¹¹ wherein R¹⁰ and R¹¹ independently from each other are selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹⁰ and R¹¹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; A is a leaving group selected from the group consisting of OR¹², SR¹² and NR¹²R¹³ wherein R¹² and R¹³ independently selected from the group consisting of C1 to C12 alkyl ; C3 to C8 cycloalkyl ; C2 to C12 alkenyl ; C2 to C12 alkynyl ; C6 to C8 aryl; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹² and R¹³ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine, preferably fluorine or chlorine ; and when Z is N⁺R¹⁰R¹¹ the positive charge is balanced by an anion, e.g. by ½ SO₄²⁻ or Cl⁻.
   with the adduct of the monosubstituted hydrazine of the formula H₂N-NHR¹ and CO₂ wherein R¹ has the meaning given above ; or
b) by reaction of a compound of formula (III) 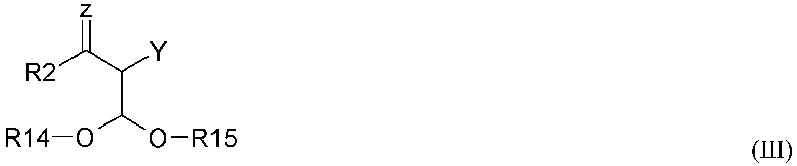 wherein R¹⁴ and R¹⁵ independently of each other are C1 to C5 alkyl groups and R², Z and Y have the meaning given above
   with the adduct of the monosubstituted hydrazine of the formula H₂N-NHR¹ and CO₂ wherein R¹ has the meaning given above.

Preferably, R¹ is C1 to C5 alkyl.

Preferably, in the context of the present invention, R² is chloromethyl, dichloromethyl, trichloromethyl, fluoromethyl, difluoromethyl, trifluoromethyl, chlorobromomethyl, chlorofluoromethyl, chlorodifluoromethyl, fluorodichloromethyl, 1-fluoroethyl, 2-fluoroethyl, 2,2-difluoroethyl, 2,2,2-trifluoroethyl, 1-chloroethyl, 2,2-dichloroethyl, 1,2-dichloroethyl, 2-chlorofluoroethyl, 2-chloro-2,2-difluoroethyl, 2,2-dichloro-2-fluoroethyl, 2,2,2-trichloroethyl, pentafluoroethyl and 1,1,1-trifluoro-prop-2-yl ; especially preferably, R² is CH₂F, CHF₂, CClF₂ or CF₃. In the most preferred fungicidal pyrazoles, R² is CHF₂. Thus, in the frame of the present invention, R² is most preferably CClF₂ or CHF₂. If R² is CClF₂, it can be reduced to CHF₂ as described in WO 2012/010692 using hydrides, Zn/alcohol or H₂/catalyst, e.g. Pd.

Preferably, Z is O. Preferably, Y is C(O)OR³ wherein R³ is a C1 to C5 alkyl group.

More preferably, R¹ is methyl or ethyl, R² is CHF₂, CClF₂ or CF₃, and R³ is methyl or ethyl.

Especially preferably, R¹ is methyl, R² is CHF₂, CClF₂ or CF₃, and R³ is methyl or ethyl.

Preferably, R³ R⁴ R⁵, R⁸, R⁹, R¹⁰, R¹¹ and R¹² are methyl, ethyl, propyl or Preferably, R³, R⁴, R⁵, R⁸, R⁹, R¹⁰, R¹¹ and R¹² are methyl, ethyl, propyl or butyl, and more preferably, they are methyl or ethyl.

Preferred pyrazole compounds are assembled in table 1.

**Table 1 : Preferred pyrazole compounds**

| |
|---|
| 3-CF₃-1-CH₃-1H-pyrazole-4-COOCH₃ |
| 3-CF₂Cl-1-CH₃-1H-pyrazole-4-COOCH₃ |
| 3-CHF₂-1 -CH₃-1H-pyrazole-4-COOCH₃ |
| 3-CF₃-1-CH₃-1H-pyrazole-4-COOCH₂CH₃ |
| 3-CF₂Cl-1-CH₃-1H-pyrazole-4-COOCH₂CH₃ |
| 3-CHF₂-1-CH₃-1H-pyrazole-4-COOCH₂CH₃ |
| 3-CF₃-1-CH₃-1H-pyrazole-4-COOCH₂CH₂CH₃ |
| 3-CF₂Cl-1-CH₃-1H-pyrazole-4-COOCH₂CH₂CH₃ |
| 3-CHF₂-1-CH₃-1H-pyrazole-4-COOCH₂CH₂CH₃ |

The 1-R¹-3-haloalkyl-pyrazole-4-carboxylic acid derivatives are precursors of fungicidal active compounds ; see for example, US 2011/0009642 and WO 03/070705.

The reaction according to step a) or step b) of the process of the invention can be performed in a solvent. Protic, aprotic, polar and nonpolar organic solvents may be used. For example, aromatic solvents, e.g. toluene, are suitable. Alcohols are also suitable as solvent, e.g. monobasic alcohols and dibasic alcohols, for example, methanol, ethanol, n-propanol, isopropanol or glycol. Hydrocarbons can be applied, for example hydrocarbons with 3 to 10 carbon atoms, e.g. n-butane, 2-methylpropane, any isomers of pentane, e.g. n-pentane, any isomers of hexane, e.g. n-hexane, any isomers of heptane, e,g, n-heptane, and any mixtures thereof. Halohydrocarbons are suitable as solvent, e.g. saturated halohydrocarbons with 1 to 10 carbon atoms wherein halo denotes chlorine and fluorine. For example, dichloromethane, trichloromethane, tetrachloromethane, trichloroethane, any isomers of pentafluoropropane, e.g. 1,1,1,3,3-pentafluoropropane, the two isomers of heptafluoropropane, e.g. 1,1,1,2,3,3,3-heptafluoropropane, any isomers of pentafluorobutane, e.g. 1,1,1,3,3-pentafluorobutane, fluorosubstituted pentanes, e.g. decafluoropentane, and and any mixtures thereof.

Advantageously, the same solvent can be used which is used in the formation of the adduct of monosubstituted hydrazine and CO₂.

If the reaction is performed according to step b), an excess of triethylorthoformiate can be applied as a solvent.

The compounds of formula (III) can be manufactured from trialkyl orthoformiate and the respective derivative of 3-oxobutyric acid having the formula (IV), R²-C(O)-CH₂-Y wherein R² and Y have the meaning given above. Preferably, in the context of the present invention, R² is chloromethyl, dichloromethyl, trichloromethyl, fluoromethyl, difluoromethyl, trifluoromethyl, chlorobromomethyl, chlorofluoromethyl, chlorodifluoromethyl, fluorodichloromethyl, 1-fluoroethyl, 2-fluoroethyl, 2,2-difluoroethyl, 2,2,2-trifluoroethyl, 1-chloroethyl, 2,2-dichloroethyl, 1,2-dichloroethyl, 2-chlorofluoroethyl, 2-chloro-2,2-difluoroethyl, 2,2-dichloro-2-fluoroethyl, 2,2,2-trichloroethyl, pentafluoroethyl and 1,1,1-trifluoro-prop-2-yl ; especially preferably, R² is CH₂F, CHF₂, CClF₂ or CF₃. Preferably, Z is O. Preferably, Y is C(O)OR³ wherein R³ is a C1 to C5 alkyl group. In view of these preferred compounds, the manufacture of the compounds of formula (IV) will be described in detail.

The preferred compounds of formula (IV) can be manufactured by reacting compounds of formula (V), R²-C(O)-CH₂-C(O)-OR³, wherein R¹ and R³ have the meaning given above, and trialkylorthoformates of formula (VI), HC(OR¹⁴)(OR¹⁵)(OR⁶) ; R¹⁴ and R¹⁵ have the meaning given above, R⁶ is C1 to C5 alkyl ; the groups R¹⁴, R¹⁵ and R⁶ may be the same or different and are preferably methyl, ethyl, propyl or butyl. More preferably, they are the same and are methyl, ethyl or n-propyl. Especially preferably, they are the same and are ethyl. Compounds of formula (IV) or (V) can be manufactured by the addition of acetic acid chlorides, preferably fluoroacetic acid chloride, difluoroacetic acid chloride, chlorodifluoroacetic acid chloride or trifluoroacetic acid chloride, to ketene as described in DE-AS 1158490 and subsequent derivatization ; the compounds of formula (V) can be obtained by the reaction of ketene with acetic acid chlorides, preferably fluoroacetic acid chloride, difluoroacetic acid chloride, chlorodifluoroacetic acid chloride or trifluoroacetic acid chloride, and subsequent reaction with an alcohol having the formula R³OH.

The respective chemical reaction between compounds of formula (V) and formula (VI) is assumed to be

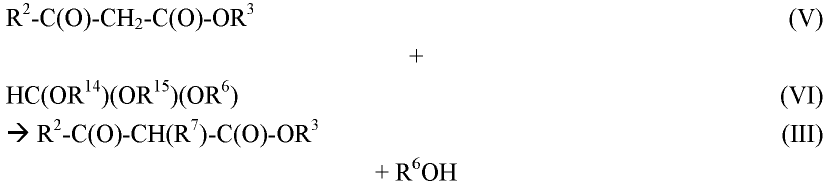

To be noted : for simplification, the reaction equation between compounds (V) and (VI) given above assumes that R¹⁴, R¹⁵ and R⁶ are the same. Otherwise, mixtures would be obtained, and different alcohols would be liberated. R⁷ is C(H)(OR¹⁴)(OR¹⁵).

The reaction between compounds of formula (V) and (VI) is performed at a temperature which allows for a reasonably fast reaction. Preferably, the temperature is equal to or greater than 80°C. More preferably, it is equal to or greater than 90°C. Especially preferably, it is equal to or greater than 100°C.

The upper limit of the reaction temperature is selected such that no undesired amounts of side reactions take place. Often, the reaction is performed at a temperature equal to or lower than 180°C, preferably, equal to or lower than 160°C.

If desired, the reaction can be performed in the presence of an inert gas, e.g. in the presence of N₂.

Contrary to reactions between compounds of formula (V) and (VI) as described in the state of the art, no carboxylic acid anhydrides, for example, no acetic acid anhydride, are applied.

If desired, the reaction between compounds of formula (V) and (VI) can be performed in the presence of one or more solvents, for example, in the presence of at least one solvent selected from the group consisting of aprotic or protic organic solvents, e.g. esters, ethers or alcohols. According to a preferred embodiment, an excess of the compound of formula (VI) is applied as solvent. Especially preferably, triethyl orthoformate is the preferred compound of formula (VI), and thus, it is the especially preferred solvent.

The reaction between compounds of formula (V) and (VI) is not sensitive to pressure ; it can be performed in a vacuum, at ambient pressure and under pressure above ambient pressure, e.g. at a pressure up to 10 bar (abs) or more. In a preferred embodiment, the resulting alcohol is distilled off, and thus, performing the reaction at ambient pressure or applying a vacuum is preferred. The distilled alcohol is of such a high purity that it can be used for other useful purposes, e.g. as a solvent or as a reagent to produce compounds of formula (V) according to the description of DE-AS 1158490.

After completion of the reaction, preferably, any solvent is removed from the reaction mixture. This is preferably performed by applying a vacuum. If, for example, triethyl orthoformate ("TEOF") is applied as starting compound of formula (VI), it is preferred to remove the excess of TEOF by keeping the reaction mixture at approximately 100°C and an initial vacuum of 100 mbar.

Solvent, e.g. trialkyl orthoformate when applied in excess, can be reused.

The resulting compound of formula (III) is obtained in sufficient purity for further reaction steps.

The reaction temperature for performing step a) or b) is very variable. For example, it can be performed between -30°C and +100°C. The reaction of step a) or of step b) is preferably performed at a temperature ranging from -20°C to 30°C.

If desired, it can be performed under a vacuum or at a pressure higher than ambient pressure, e.g. up to 20 bar (abs), but the reaction of step a) or of step b) is preferably performed at ambient pressure. An inert gas, e.g. nitrogen, can be used to prevent the intrusion of air or moisture.

After removing the solvent, e.g. by applying a vacuum, pure pyrazole product is obtained. The separation of the 3-R² isomer from the 5-R² isomer can be achieved by methods known in the art, especially by distillation in a vacuum.

The manufacture of the pyrazoles explained above is a preferred embodiment. The adduct of monosubstituted hydrazine and CO₂ may also be used in other reactions where monosubstituted hydrazine is applied. For example, it can be used as "safe form" of monosubstituted hydrazines for all type of chemical reactions where normal pure hydrazine or its solution in water or other solvents is applied, like condensation with carbonyl compounds, alkylations, reactions with esters, acid chlorides or anhydrides to form hydrazides.

The advantage of using the CO₂ adduct instead of the monosubstituted hydrazine is that exothermic condensation is avoided. It is assumed that ring formation according to reaction a) or b) occurs without intermediate formation of a 2-acylated or 2-iminoalkylated acrylic acid. Another advantage is the higher selectivity in formation of the 3-R² isomer over the unwanted 5-R² isomer.

Another aspect of the present invention is the use of the adduct of monosubstituted hydrazine of the formula H₂N-NHR¹ and CO₂ wherein R¹ has the meaning given above, be it neat, dissolved, emulgated or suspended in a solvdent,as a substitute for H₂N-NHR¹ or hydrazones of the formula (R⁸)(R⁹)C=N-NHR¹ wherein R⁸ and R⁹ independently selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁸ and R⁹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; X is fluorine, chlorine, bromine and iodine, preferably fluorine or chlorine. Here, R⁸ and R⁹ preferably are methyl or ethyl, especially both are methyl.

Still another aspect of the present invention are solutions, suspensions and emulsions of the adduct of hydrazine, substituted by one R¹ group, and CO₂ in an organic solvent. R¹ has the meaning given above, and is preferably methyl, ethyl, n-propyl, i-propyl, n-butyl, s-butyl, t-butyl or n-pentyl ; especially preferably, R¹ is methyl or ethyl, most preferably, R¹ is methyl. As solvent, aprotic, protic, polar and nonpolar solvents are suitable. For example, aromatic solvents, e.g. toluene, are suitable. Alcohols are also suitable as solvent, e.g. monobasic alcohols and dibasic alcohols, for example, methanol, ethanol, n-propanol, isopropanol or glycol. Hydrocarbons can be applied, for example hydrocarbons with 3 to 10 carbon atoms, e.g. n-butane, 2-methylpropane, any isomers of pentane, e.g. n-pentane, any isomers of hexane, e.g. n-hexane, any isomers of heptane, e,g, n-heptane, and any mixtures thereof. Halohydrocarbons are suitable as solvent, e.g. saturated halohydrocarbons with 1 to 10 carbon atoms wherein halo denotes chlorine and fluorine. For example, dichloromethane, trichloromethane, tetrachloromethane, trichloroethane, any isomers of pentafluoropropane, e.g. 1,1,1,3,3-pentafluoropropane, the two isomers of heptafluoropropane, e.g. 1,1,1,2,3,3,3-heptafluoropropane, any isomers of pentafluorobutane, e.g. 1,1,1,3,3-pentafluorobutane, fluorosubstituted pentanes, e.g. decafluoropentane, and any mixtures thereof.

The concentration of the adduct of the suspended monosubstituted hydrazine and CO₂ in the solvent may be between 1 % by weight to 99 % by weight. Preferably, it is between 3 to 25 % by weight, more preferably, it is 5 to 10 % by weight.

The suspensions are suitable for the manufacture of, e.g., the pyrazoles substituted in the 1-position by an R¹ group as described above.

Should the disclosure of any patents, patent applications, and publications which are incorporated herein by reference conflict with the description of the present application to the extent that it may render a term unclear, the present description shall take precedence.

The following examples are intended to explain the invention in further detail without the intention to limit it.

### General remarks :

Triethylorthoformate (TEOF) is commercially available. Ethyl difluorochloroacetic acid (ECDFAA) is commercially available from Solvay Fluor GmbH, Hannover /Deutschland.

### Example 1 : Manufacture of 4-Chloro-2-diethoxymethyl-4,4-difluoro-3-oxobutyric acid ethyl ester ("ECDFAA-DEM")

TEOF (130 g, 0.88 mol) was placed in a flask equipped with a magnetic stirring bar and a vacuum distillation cooler. ECDFAA (35 g, 0.175 mol) was added.

The resulting mixture was heated to 110°C (outer temperature of the flask) under a mild vacuum (300 mbar) ; ethanol formed during the reaction is slowly distilled out of the vessel. After 2h, the starting material was completely consumed, and product formation was observed by GC analysis. Excess of TEOF was removed from the reaction mixture by distillation (at 100°C, starting with a pressure of 100 mbar at the beginning and 10 mbar at the end, to remove all TEOF traces), resulting pure product as pale yellow liquid with quantitative yield.

¹H NMR (500 MHz, CHLOROFORM-*d*) δ ppm 1.15 (t, 3 H) 1.20 (t, 3 H) 1.27 (t, 3 H) 3.49 - 3.57 (m, 1H) 3.61 - 3.69 (m, 1H) 3.71 - 3.79 (m, 2 H) 4.23 (q, 2 H) 4.34 (d, *J*=8.24 Hz, 1 H) 5.18 (d, *J*=8.24 Hz, 1 H). *Example 2 :* Manufacture of 4-chloro-2-[1-ethoxy-meth-(Z)-ylidene]-4,4-difluoro-3-oxo-butyric acid ethyl Ester ("EME-CDFAA")

Triethylorthoformate (415 g = 2.15 mol), ECDFAA (280 g = 1.4 mol) and acetic acid anhydride (428 g = 4.2 mol) were given into a flask equipped with a vacuum distillation cooler. The resulting mixture was heated to 135°C (outer temperature. Slowly, formed light boilers were distilled off. After 9 hours, the mixture was heated to 110°C under slowly lowering the pressure from ambient pressure to a vacuum of 10 mbar ; all light boilers were distilled out of the reaction mixture. A yellow-brown liquid with a purity of 87 % (determined by gas chromatography) remained in the flask. The raw product could be used immediately without further purification for the next reaction step.

¹H NMR (500 MHz, CHLOROFORM-*d*), δ ppm (the spectrum shows a ratio of E to Z compound of 1:2.5) : 1.25 - 1.35 (4 t, 6 H) ; 4.20 - 4.38 (4 q, 4 H) and 7.76 (bs, 1 H).

### Example 3 : Manufacture of an adduct of monomethyl hydrazine and CO₂

### 3.1. Reaction performed in toluene

Monomethyl hydrazine ("MMH" ; 82.8 mg = 1.8 mmol) was dissolved in 10 ml toluene and then an excess of dry ice was added under stirring. The solvent was removed, and a colorless sticky plastic-like solid remained in the flask.

¹H NMR (500 MHz, DMSO-*d6*), δ ppm : 2.52 (bs, 3H, CH3), 6.38 (bs, 3H, NHₓ)

### 3.2. Reaction in other solvents

Example 3.1 was repeated, but using HFC-1,1,1,3,3-pentafluorobutane, ethanol or hexane as solvent. A white suspension of the formed adduct in the solvent was observed.

### Example 4 : Manufacture of ethyl 1-methyl-3-chlorodifluoromethyl-pyrazole-4-carboxylate ("CDFMMP")

The emulsion of the adduct of MMH-CO₂ in 10 ml toluene was cooled to -10°C, and then EME-CDFAA (0.46 g = 1.8 mmol), dissolved in 2 ml toluene, was added drop wise. The resulting mixture was stirred for 2 h at -10°C, allowed to warm up to room temperature, and stirred for further 2 h. The solvent was evaporated, yielding a pure mixture of the isomers of CDFMMP with 1-methyl substitution (target compound) and 5-methyl (unwanted side product) in a ratio of 90.1 to 8.8 (determined by gas chromatography).

The desired ethyl 1-methyl-3-chlorodifluoromethyl-pyrazole-4-carboxylate can be isolated by distillation (boiling point 100 - 102°C/0.15 mbar) from the unwanted 5-isomer (boiling point 70 to 80°C/0.15 mbar).

### Example 5 : Manufacture of ethyl 1-methyl-3-chlorodifluoromethyl-pyrazole-4-carboxylate from 4-Chloro-2-diethoxymethyl-4,4-difluoro-3-oxobutyric acid ethyl ester and the adduct of MMH-CO₂

Example 4 is repeated, but instead of EME-CDFAA, 4-Chloro-2-diethoxymethyl-4,4-difluoro-3-oxo-butyric acid ethyl ester is used as starting compound, using the same conditions like in example 4, except for the post-stirring time at room temperature which was extended to overnight.

The ethyl 1-methyl-3-chlorodifluoromethyl-pyrazole-4-carboxylate formed in the examples can be reduced with Zn or other reducing agents to provide ethyl 1-methyl-3-difluoromethyl-pyrazole-4-carboxylate which is an intermediate for fungicides.

## Claims

1. A process for the manufacture of heterocyclic compounds having a heterocyclic ring with an =N-N(R¹) group in the ring wherein R¹ is an organic substituent wherein a hydrazinium-CO₂ compound, monosubstituted by an R¹ group, is applied to introduce the =N-N(R¹) group into the heterocyclic ring.

2. The process of claim 1 wherein R¹ is an organic substituent selected from the group consisting of C1 to C12 alkyl ; C3 to C8 cycloalkyl ; C2 to C 12 alkenyl ; C2 to C12 alkynyl ; C6 to C8 aryl ; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine, preferably fluorine or chlorine.

3. The process of claim 1 or 2 wherein the hydrazinium-CO₂ compound, monosubstituted by an R¹ group, is reacted with a compound having a C=O group, a C=S group or an imido group in α position to
a) a C=C-A group wherein A is a leaving group selected from the group consisting of OR¹², SR¹² and NR¹²R¹³ wherein R¹² and R¹³ independently selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹² and R¹³ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group, and X is fluorine, chlorine, bromine and iodine, preferably fluorine or chlorine ; or
b) a CH-C(OR¹⁴)(OR¹⁵) group wherein R¹⁴ and R¹⁵ independently of each other are C1 to C5 alkyl groups.

4. The process of anyone of claims 1 to 3 for the manufacture of 1-alkyl-3-haloalkyl-pyrazole-4-carboxylic acid derivatives of formula (I) 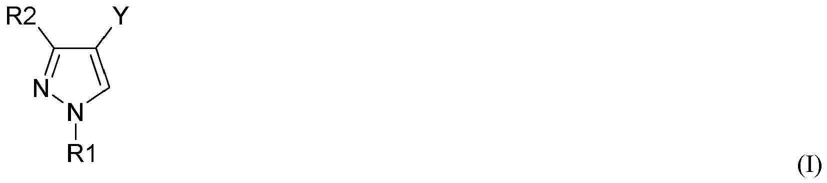 In which R¹ has the meaning given above ; R² is selected from C1 to C4 alkyl groups which may be substituted by one, two or three halogen atoms selected from the group consisting of F, Cl and Br or a CF₃ group ;
Y is selected from the group consisting of C(O)OR³, CN and C(O)NR⁴R⁵ wherein R³, R⁴ and R⁵ are independently of each other are selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁴ and R⁵ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine, preferably fluorine or chlorine ;
a) by reaction of a 2-acylated or 2-iminoalkylated acrylic acid derivative of the formula (II) 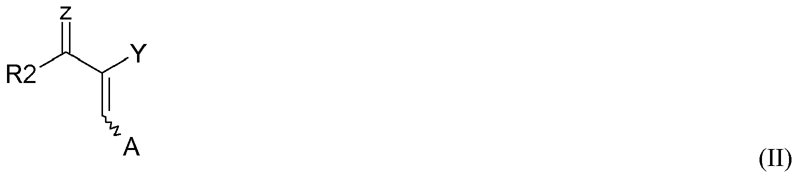 in which Z is selected from the group consisting of O, S and N⁺R¹⁰R¹⁰ wherein R¹⁰ and R¹¹ independently from each other are selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹⁰ and R¹¹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; A is a leaving group selected from the group consisting of OR¹², SR¹² and NR¹²R¹³ wherein R¹² and R¹³ independently selected from the group consisting of C1 to C12 alkyl ; C3 to C8 cycloalkyl ; C2 to C 12 alkenyl ; C2 to C 12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹² and R¹³ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C 12 group ; and X is fluorine, chlorine, bromine and iodine, preferably fluorine or chlorine ; and when Z is N⁺R¹⁰R¹¹ the positive charge is balanced by an anion ;
with the adduct of the monosubstituted hydrazine of the formula H₂N-NHR¹ and CO₂ wherein R¹ has the meaning given above ; or
b) by reaction of a compound of formula (III) 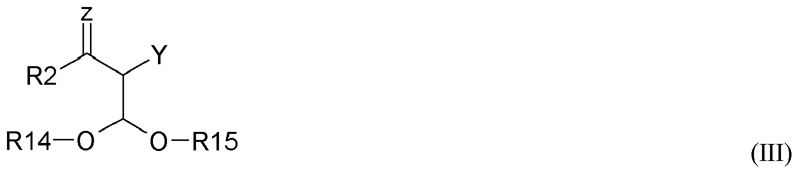 wherein R¹⁴ and R¹⁵ independently of each other are C1 to C5 alkyl groups and R², Z and Y have the meaning given above
with the adduct of the monosubstituted hydrazine of the formula H₂N-NHR¹ and CO₂ wherein R¹ has the meaning given above.

5. The process of anyone of claims 1 to 4 wherein R¹ is C1 to C5 alkyl ; or C1 to C5 alkyl, substituted by at least one halogen atom.

6. The process of anyone of claims 1 to 5 wherein Z is O.

7. The process of anyone of claims 1 to 6 wherein Y is C(O)OR³ wherein R³ is a C1 to C5 alkyl group.

8. The process of anyone of claims 1 to 7 wherein R² is selected from the group consisting of CH₂F, CHF₂, CClF₂ or CF₃.

9. The process of anyone of claims 1 to 7 wherein the hydrazinium-CO₂ compound, monosubstituted by an R¹ group, is introduced into the reaction in the form of a solution in an aromatic solvent.

10. The process of claim 9 wherein the aromatic solvent is toluene.

11. The process of anyone of claims 4 to 10 wherein R² is CClF₂ and is reduced to form CHF₂.

12. A solution, emulsion or suspension of a hydrazinium-CO₂ compound, monosubstituted by an R¹ group, wherein R¹ is an organic substituent, in an organic solvent.

13. The solution, emulsion or suspension of claim 12 R¹ is C1 to C5 alkyl ; or C1 to C5 alkyl, substituted by at least one halogen atom.

14. The emulsion of claims 12 or 13 wherein the solvent is toluene.

15. The suspension of claims 12 or 13 wherein the solvent is a saturated halohydrocarbons with 1 to 10 carbon atoms wherein halo denotes chlorine and fluorine, especially dichloromethane, trichloromethane, tetrachloromethane, trichloroethane, any isomers of pentafluoropropane, especially 1,1,1,3,3-pentafluoropropane, the two isomers of heptafluoropropane, especially 1,1,1,2,3,3,3-heptafluoropropane, any isomers of pentafluorobutane, especially 1,1,1,3,3-pentafluorobutane, or fluorosubstituted pentanes, especially decafluoropentane ; and any mixtures thereof.

16. Use of a hydrazinium-CO₂ compound, monosubstituted by an R¹ group, as a substitute for H₂N-NHR¹ wherein R¹ is an organic substituent selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group, and X is fluorine, chlorine, bromine and iodine, preferably fluorine or chlorine ; or hydrazones of the formula (R⁸)(R⁹)C=N-NHR¹ wherein R⁸ and R⁹ independently selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁸ and R⁹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; X is fluorine, chlorine, bromine and iodine, preferably fluorine or chlorine ;
in the manufacture of organic heterocyclic compounds.
